# EUROPEAN PATENT SPECIFICATION

(11) **EP 0 394 448 B1**
(45) Date of publication and mention of the grant of the patent: **01.05.1996**
(21) Application number: 89902080.4
(22) Date of filing: 07.02.1989
(51) Int. Cl.: C12P 7/42

(54) **PROCESS FOR PREPARING OPTICALLY ACTIVE 2-HYDROXY-4-PHENYLBUTYRIC ACIDS**
VERFAHREN ZUR HERSTELLUNG OPTISCH AKTIVER 2-HYDROXY-4-PHENYLBUTANSÄURE
PROCEDE DE PREPARATION D'ACIDES 2-HYDROXY-4-PHENYLBUTYRIQUES OPTIQUEMENT ACTIFS

(30) Priority: 08.02.1988 JP 25738/88; 28.04.1988 JP 105893/88; 28.04.1988 JP 105894/88
(43) Date of publication of application: 31.10.1990
(73) Proprietor: DAICEL CHEMICAL INDUSTRIES, LTD., Sakai-shi Osaka-fu 590 (JP)
(72) Inventor: MATSUYAMA, Akinobu, Arai-shi Niigata 944 (JP); NIKAIDO, Teruyuki, Arai-shi Niigata 944 (JP); KOBAYASHI, Yoshinori, Joetsu-shi Niigata 942 (JP)
(74) Representative: Grünecker, Kinkeldey, Stockmair & Schwanhäusser Anwaltssozietät
(86) International application number: JP8900120
(87) International publication number: WO8907147

(56) References cited:
- EP-A- 0 024 547
- EP-A- 0 130 288
- EP-A- 0 137 301
- EP-A- 0 218 863
- EP-A- 0 347 374
- JP-B- 26 335

## Description

### Technical Field

This invention relates to a process for the production of optically active 2-hydroxy-4-phenylbutyric acid. More particularly, it relates to a process for the production of 2-hydroxy-4-phenylbutyric acid which comprises treating 2-oxo-4-phenylbutyric acid with a microorganism, which has been optionally treated, capable of asymmetrically reducing 2-oxo-4-phenylbutyric acid into either (R)-2-hydroxy-4-phenylbutyric acid or (S)-2-hydroxy-4-phenylbutyric acid and recovering the (R)-2-hydroxy-4-phenylbutyric acid or (S)-2-hydroxy-4-phenylbutyric acid thus formed.

Optically active 2-hydroxy-4-phenylbutyric acid is an important intermediate in the synthesis of various drugs, for example, a remedy for hypertension.

### Background Art

Known methods for the production of optically active 2-hydroxy-4-phenylbutyric acid include optical resolution of an ester prepared from racemic 2-hydroxy-4-phenylbutyric acid and ℓ-menthol [cf. D. Biquard, Ann. de. Chimie, 20, 146 (1933)] and chemical synthesis from benzylmagnesium chloride and optically active glycidic acid (cf. JP-A-212329/1987). However the former method comprising the optical resolution is disadvantageous in that the ℓ-menthol is expensive, while the latter method comprising the chemical synthesis is disadvantageous in that optically active serine which is the starting material for the preparation of the optically active glycidic acid is expensive for industrial uses.

Although there have been reported microbial enzymes capable of asymmetrically reducing a 2-oxocarboxylic acid to thereby give an optically active 2-hydroxy carboxylic acid (cf. JP-A-12975/1985, JP-B-11591/1986, JP-A-100286/ 1987, JP-A-32480/1988 and JP-A-32493/1988). However none of these references discloses a process for the production of optically active 2-hydroxy-4-phenylbutyric acid from 2-oxo-4-phenylbutyric acid. EP-A-0 137 301 discloses the enzyme 2-oxo-carboxylic acid reductase which can be obtained from the microorganisms of the genera Proteus and which enzyme can be used for the reduction of 2-oxo-carboxylic acids.

Furthermore, no process for the production of optically active 2-hydroxy-4-phenylbutyric acid from 2-oxo-4-phenylbutyric acid by using a microorganism has been reported so far.

### Disclosure of Invention

The present inventors have paid attention to a process for conveniently producing optically active 2-hydroxy-4-phenylbutyric acid having a high optical purity through asymmetric reduction with a microorganism and have attempted to find out microorganisms suitable for the above purpose. As a result, they have found that microorganisms capable of producing (R)-2-hydroxy-4-phenylbutyric acid selected from among those belonging to the genera Streptococcus, Sporolactobacillus, Pediococcus, Saccharomycopsis, Saccharomyces, Rhodotolura, Candida, Torulaspora, Sporidiobolus, Ambrosiozyma, Arthroascus, Botryoascus, Clavispora, Debaryomyces, Lipomyces, Lodderomyces, Metschnikowia, Geotrichum, Kluyveromyces, Cryptococcus, Trigonopsis, Wickerhamia, Wickerhamiella, Schizosaccharomyces , Rhodosporidium, Stephanoascus, Hansenula, Achromobacter, Bacillus, Escherichia, Micrococcus, Serratia, Staphylococcus, Mycobacterium, Brevibacterium, Chromobacterium, Aureobacterium, Flavobacterium, Salmonella, Erwinia, Agrobacterium, Acetobacter, Paracoccus, Protaminobacter, Pseudomonas, Vibrio, and microorganisms capable of producing (S)-2-hydroxy-4-phenylbutyric acid selected from among those belonging to the genera Pediococcus, Streptococcus, Brevibacterium and Corynebacterium can be used.

Especially preferred embodiments of the present invention are mentioned in claims 2 and 3.

Particular examples of the microorganism capable of producing (R)-2-hydroxy-4-phenylbutryic acid from 2-oxo-4-phenylbutyric acid include Streptococcus faecalis IFO12964, Streptococcus faecium NRIC1145 and ATCC19434, Sterptococcus sp. IFO3427, Streptococcus sp. IFO3535, Streptococcus lactis AHU1089, Streptococcus uberis NRIC1153 and ATCC19436, Sporolactobacillus inulinus NRIC1133 and ATCC15538, Pediococcus acidilactici NRIC1102 and ATCC8081, Saccharomycopsis fibuligera IFO0103, Saccharomycopsis lipolytica IFO1550, Saccharomyces bayanus IFO0262, Saccharomyces kluyveri KFO1894, Saccharomyces uvarum IFO0565, Saccharomyces chevalieri IFO0222, Rhodotolura glutinis AHU3942, Candida humicola IFO0760, Candida parapsilosis LFO1396, Candida rugosa IFO0750, Torulaspora delbruekii IFO0955, Sporidiobolus johnsonii IFO6903, Ambrosiozyma cicatricosa IFO1846, Ambrosiozyma platypodis LFO1471, Arthroascus javanensis LFO1848, Botryoassus synnaedendrus IFO1604, Clavispora lusitanias IFO1019, Debaryomyces hansenii IFO0083, Lipomyces starkeyi IFO1289, Lodderomyces elongisporus IFO1676, Metschnikowia bicuspidata IFO1408; Geotrichum candidum IFO4601, Kluyveromyces lactis IFO1903, Cryptococcus neoformans IAM4788, Trigonopsis variabilis IFO0755, Wickerhamia fluorescens IFO1116, Wickerhamiella domercquii IFO1857, Schizosaccharomyces octosporus IFO0353, Rhodosporidium dibovatum IFO1829 , Stephanoascus ciferrii IFO1854, Hansenula fabianii IFO1253, Achromobacter pestifer ATCC23584, Bacillus licheniformis IFO12200, Escherichia coli IFO3544, Micrococcus luteus IFO12992, Serratia marcescens IAM12143, Staphylococcus aureus IFO3060, Mycobacterium smegmatis IFO3153, Brevibacterium iodinum IFO3558, Aureobacterium testaceum IFO12675, Flavobacterium suaveolens IFO3752, Salmonella typhimurium IFO12529, Erwinia carotovora IFO3830, Agrobacterium radiobacter IFO12664, Acetobacter pasteurianus ATCC10245, Paracoccus denitrificans IFO12442, Protaminobacter ruber IAM9081, Pseudomonas aureofaciens IFO3522 and Vibrio anguillarum IFO12710.

Examples of the microorganism capable of producing (S)-2-hydroxy-4-phenylbutyric acid from 2-oxo-4-phenylbutyric acid include Pediococcus pentosaceus IFO3891, Streptococcus agalactiae NRIC1137 and ATCC13813, Streptococcus lactis NRIC1149 and ATCC19435, Brevibacterium ammoniagenes IAM1641 and Corynebacterium glutamicum ATCC13032.

Each strain may be either a wild type, a variant or a recombinant obtained by genetic engineering such as cell fusion or gene recombination.

Microorganisms having IFO numbers assigned thereto are described in the List of Culture, 8th ed., vol. 1 (1988) published by the Institute for Fermentation, Osaka (IFO) and available therefrom. Those having AHU numbers are described in the Catalogue Culture, 4th ed. (1987) published by Japan Federation of Culture Collection (JFCC) and available from the Faculty of Agriculture, Hokkaido University. Those having ATCC numbers are described in the Catalogue of Bacteria Phages rDNA Vectors, 16th ed. (1985) published by American Type Culture Collection (ATCC) and available therefrom. Those having NRIC numbers are described in the Culture Collection of NODAI No. 1 (1985) published by Tokyo University of Agriculture and available therefrom. Those having IAM numbers are available from the Institute of Applied Microbiology, the University of Tokyo.

The microorganism to be used in the present invention may be cultured in any medium so long as it can grow therein. Any carbon source may be used so long as said microorganism can utilize it. Examples thereof include sugars such as glucose, fructose, sucrose and dextrin, alcohols such as sorbitol, ethanol and glycerol, organic acids such as fumaric, citric, acetic and propionic acids and salts thereof, hydrocarbons such as paraffin and mixtures thereof. Examples of a nitrogen source include ammonium salts of inorganic acids, such as ammonium chloride, ammonium sulfate and ammonium phosphate, ammonium salts of organic acids, such as ammonium fumarate and ammonium citrate, nitrogenous materials such as meat extract, yeast extract, corn steep liquor, casein hydrolysate and urea and mixtures thereof. Furthermore various nutritional sources commonly used in the culture of microorganisms, such as inorganic salts, trace metal salts and vitamins, may be appropriately mixed and used in the present invention. In addition, materials effective in promoting the growth of the microorganism, in elevating the productivity of the target compound or in maintaining the pH value of the medium on the desired level may be added, if required.

The pH value of the medium may be adjusted to 3.0 to 9.5, preferably 4 to 8. The culture may be carried out at a temperature of 20 to 45°C, preferably 25 to 37°C, either aerobically or anaerobically under conditions suitable for the growth of the microorganism for 15 to 120 hours, preferably 12 to 72 hours.

The reduction may be effected by using the culture medium as such. Alternately, the cells may be separated by, for example, centrifugation, optionally washed and resuspended in a buffer solution or water. Then 2-oxo-4-phenylbutyric acid may be added to the suspension thus obtained. In this reaction, it is sometimes preferable to add a carbon source such as glucose or sucrose to the medium to thereby supply energy. The cells may be used as such in the form of viable cells. Alternately, they may be ground, treated with acetone or lyophilized. These cells, which have been optionally treated, may be immobilized prior to the use by a conventional method such as the polyacrylamide gel, carrageenan gel, alginic acid gel or agar gel method. Furthermore, an enzyme obtained from said treated cells by combining known methods may be used in the present invention.

The 2-oxo-4-phenylbutyric acid may be added either at once at the initiation of the reaction or by portions either as such, dissolved in water or an inert organic solvent or dispersed in, for example, a surfactant. The 2-oxo-4-phenylbutyric acid may be used in the form of various salts such as ammonium, sodium, calcium or potassium salt.

The reaction may be conducted at a pH value of 3 to 9, preferably 5 to 8, at 10 to 60°C, preferably 20 to 40°C for 1 to 120 hours with or without stirring. The concentration of the substrate may preferably range from 0.1 to 10%, though it is not restricted thereby.

The optically active 2-hydroxy-4-phenylbutyric acid thus formed may be readily collected by extracting the reaction mixture, from which the cells may be optionally separated, with an organic solvent and purifying the extract by, for example, column chromatography or recrystallization.

### Best Mode for Carrying Out the Invention

To further illustrate the present invention, the following Examples will be given.

In each Example, the absolute configuration and optical purity were determined by extracting the reaction product with ethyl acetate, ethylesterifying the same in a conventional manner and subjecting the obtained ester to high performance liquid chromatography by using an optical resolution column [column: Chiral cell OB^{®}, mfd, by Daicel Chemical Industries, Ltd., 4.6 mm (i.d.) x 250 mm, solvent: n-hexane : 2-propanpol (19 : 1 v/v), flow rate: 0.5 ml/min, Detection: 254 nm]. The reaction yield was determined by high performance liquid chromatography by using a reverse phase column [column: Nucleosil 10C18, 4.0 mm (i.d.) x 250 mm, solvent: 40 mM phosphate buffer (pH 3.0)/acetonitrile = 4 : 1, flow rate: 1 ml/min, detection: 254 nm].

### Example 1

100 ml of a medium comprising 2% of glucose, 0.5% of yeast extract, 0.5% of peptone, 0.5% of meat extract, 0.2% of dipotassium phosphate and 1% of calcium carbonate was introduced into a 500-ml Erlenmeyer flask and sterilized. Next, each strain specified in Table 1 was inoculated thereto and cultured therein at 30°C for 30 hours under shaking.

After the completion of the culture, the cells were separated by centrifugation and washed once with a physiological saline solution to thereby give viable cells.

7.5 ml of distilled water was introduced into a 100-ml Erlenmeyer flask and the above viable cells were suspended therein. 1.2 g of glucose was added to the suspension thus obtained and the resulting mixture was shaken under rotation at 30° for 10 minutes. Then 2.5 ml of a 4% aqueous solution of 2-oxo-4-phenylbutyric acid, with the pH value adjusted to 7 with caustic potash, and 0.3 g of calcium carbonate were added thereto and the resulting mixture was shaken under rotation at 30°C for 40 hours.

After the completion of the reaction, sulfuric acid was added to the reaction mixture to thereby adjust the pH value thereof to 1 or below. Then the optically active 2-hydroxy-4-phenylbutyric acid thus formed was extracted with 20 ml of ethyl acetate. The yield was determined by removing a given amount of the solvent from the ethyl acetate phase under reduced pressure, dissolving the residue thus obtained in the abovementioned eluent and determining the 2-hydroxy-4-phenylbutyric acid thus formed by high performance liquid chromatography by using a reverse phase column.

Similarly, a given amount of the solvent was removed from the ethyl acetate phase under reduced pressure and the residue was ethyl-esterified in a conventional manner. Then the absolute configuration and optical purity of the 2-hydroxy-4-phenylbutyric acid thus formed was determined by high performance liquid chromatography by using an optical resolution column.

Table 1 shows the results.

### Example 2

100 ml of a YM medium comprising 0.3% of yeast extract, 0.3% of malt extract, 0.5% of peptone and 2% of glucose (pH 6.0) was introduced into a 500-ml Sakaguchi flask and sterilized. Then each strain specified in Table 2 was inoculated thereto and cultured therein at 30°C for 48 hours under shaking.

After the completion of the culture, the cells were separated by centrifugation and washed once with a physiological saline solution to thereby give viable cells.

7.5 ml of distilled water was introduced into a 100-ml Erlenmeyer flask and the abovementioned viable cells were suspended therein. Then 1.2 g of sucrose was added to the obtained suspension and the mixture was reciprocally shaken at 30°C for 10 minutes. Next, 2.5 ml of a 4% aqueous solution of 2-oxo-4-phenylbutyric acid, with the pH value adjusted to 7 with caustic potash, was added thereto and the resulting mixture was shaken under rotation at 30°C for 20 hours.

After the completion of the reaction, the reaction mixture was treated in the same manner as the one described in Example 1. Thus the reaction yield and optical purity of the product were determined.

Table 2 shows the results.

### Example 3

Each microorganism specified in Table 3 was subjected to the same treatment as that described in Example 1 except that the medium contained no calcium carbonate and had a pH value of 7 and that the culture was reciprocally conducted in a 500-ml Sakaguchi flask.

Table 3 shows the results.

### Example 4

### Isolation of (R)-2-hydroxy-4-phenylbutyric acid

After the completion of the reaction, the pH value of the reaction mixture was adjusted to 1.5 with conc. sulfuric acid. The (R)-2-hydroxy-4-phenylbutyric acid thus formed was extracted with 200-ml portions of ethyl acetate twice. The ethyl acetate phase was dehydrated with anhydrous Glauber's salt and the solvent was removed under reduced pressure to thereby give crude crystals.

These crystals were recrystallized from toluene.

### Example 5

Wickerhamiella domercquii IFO1857 was inoculated into 2-ℓ of the same medium as the one described in Example 3 in a 5-ℓ jar fermenter and cultured therein at 30°C under stirring at 400 rpm and aerating at 1 vvm for 30 hours.

After the completion of the culture, the cells were collected by centrifugation and washed with 1-ℓ of water. Then these cells were suspended in 200 ml of water and introduced into a 1-ℓ Erlenmeyer flask. 20 ml of a 10% aqueous solution of 2-oxo-4-phenylbutyric acid, with the pH value adjusted to 7 with caustic potash, and 20 g of glucose were added thereto and the obtained mixture was allowed to react at 30°C under stirring for 24 hours.

After the completion of the reaction, the reaction mixture was treated in the same manner as the one described in Example 4. Thus 1.2 g of the aimed (R)-2-hydroxy-4-phenylbutyric acid was obtained in the form of crystals (yield: 60%, optical purity: 99% e.e.).

### Example 6

Flavobacterium suaveolens IFO3752 was inoculated into 2-ℓ of the same medium as the one described in Example 3 in a 5-ℓ jar fermenter and cultured therein at 30°C under stirring at 400 rpm and aerating at 1 vvm for 30 hours.

After the completion of the culture, the cells were collected by centrifugation and washed with 1-ℓ of water. Then these cells were suspended in 400 ml of water and introduced into a 2-ℓ Erlenmeyer flask. 50 ml of a 10% aqueous solution of 2-oxo-4-phenylbutyric acid, with the pH value adjusted to 7 with caustic potash, and 40 g of glucose were added thereto and the obtained mixture was allowed to react at 30°C under stirring for 72 hours.

After the completion of the reaction, the reaction mixture was treated in the same manner as the one described in Example 4. Thus 2.3 g of crude crystals of the aimed (R)-2-hydroxy-4-phenylbutyric acid were obtained. After recrystallization from toluene, 1.8 g of the aimed (R)-2-hydroxy-4-phenylbutyric acid was obtained in the form of crystals (yield: 36%, optical purity: 99% e.e.).

### Example 7

Streptococcus agalactiae NRIC1137 was inoculated into 2-ℓ of the same medium as the one described in Example 3 in a 5-ℓ jar fermenter and cultured therein at 30°C under stirring at 100 rpm for 30 hours.

After the completion of the culture, the cells were collected by centrifugation and washed with 1-ℓ of water. Then these cells were suspended in 200 ml of water and introduced into a 1-ℓ Erlenmeyer flask. 25 ml of a 10% aqueous solution of 2-oxo-4-phenylbutyric acid, with the pH value adjusted to 7 with caustic potash, 20 g of glucose and 2 g of calcium carbonate were added thereto and the obtained mixture was allowed to react at 30°C under stirring for 24 hours.

After the completion of the reaction, the reaction mixture was treated in the same manner as the one described in Example 4. Thus 1.4 g of crude crystals of the aimed (S)-2-hydroxy-4-phenylbutyric acid was obtained. After recrystallization from toluene, 1.1 g of the aimed (S)-2-hydroxy-4-phenylbutyric acid was obtained in the form of crystals (yield: 44%, optical purity: 98% e.e.).

The process of the present invention for the production of optically active 2-hydroxy-4-phenylbutyric acid through asymmetric reduction by using a microorganism makes it possible to readily produce optically active 2-hydroxy-4-phenylbutyric acid having a high optical purity. Thus it is highly advantageous as an industrial process.

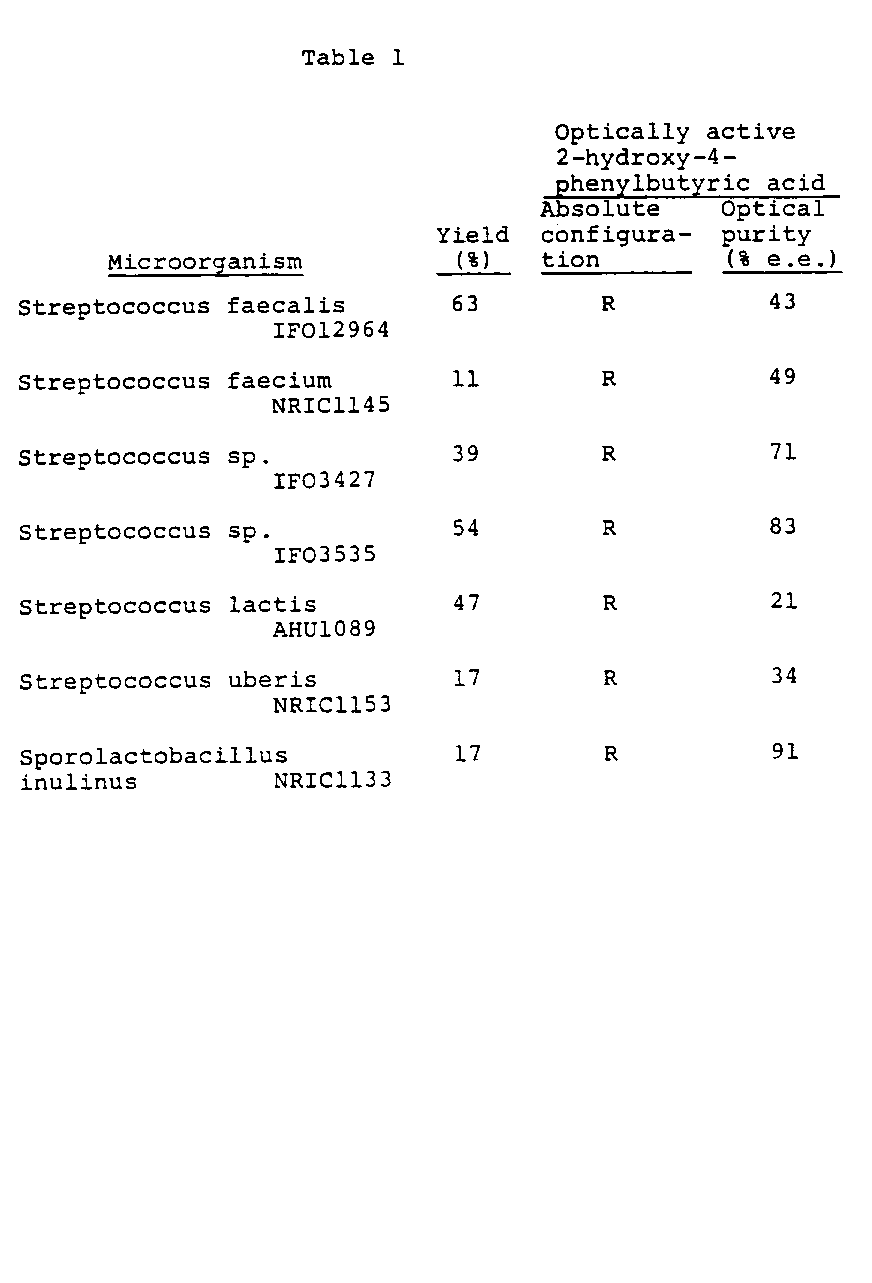

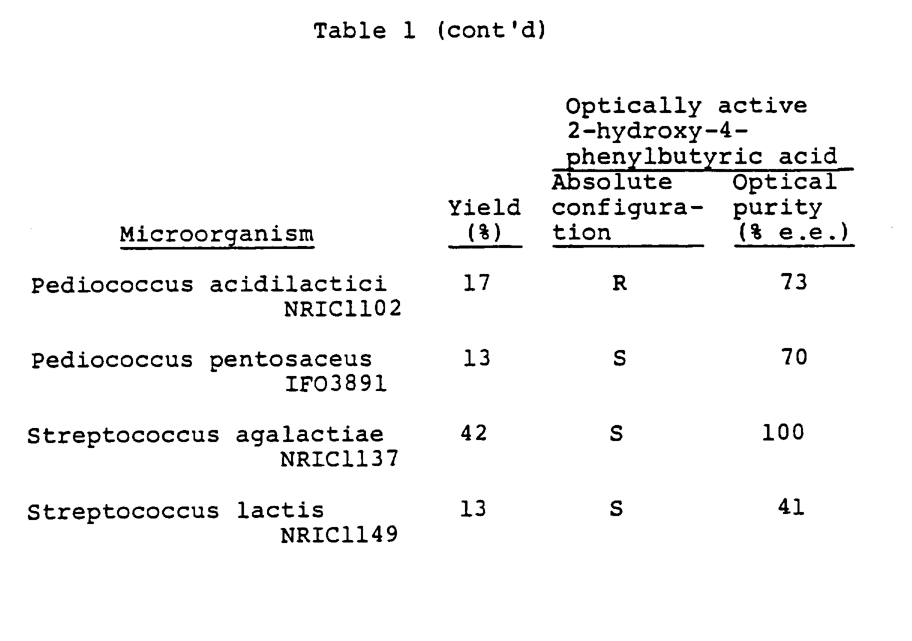

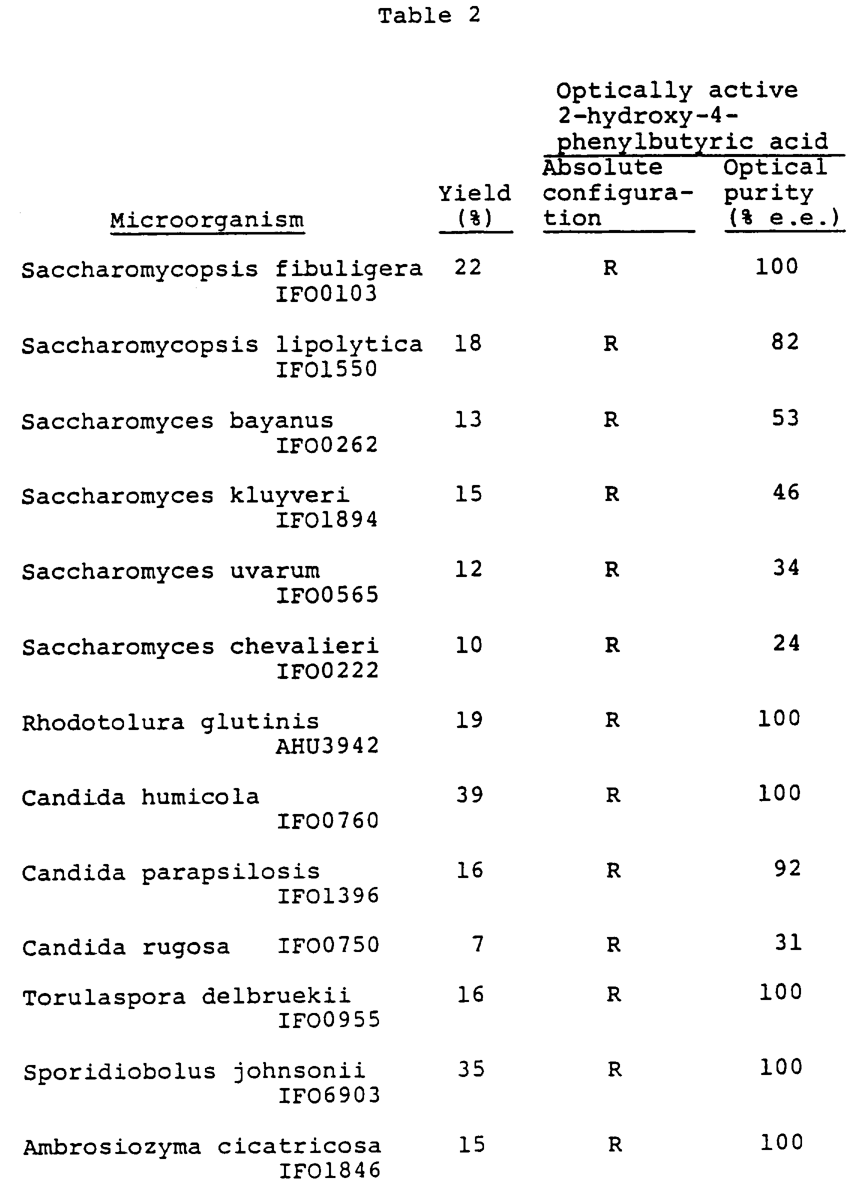

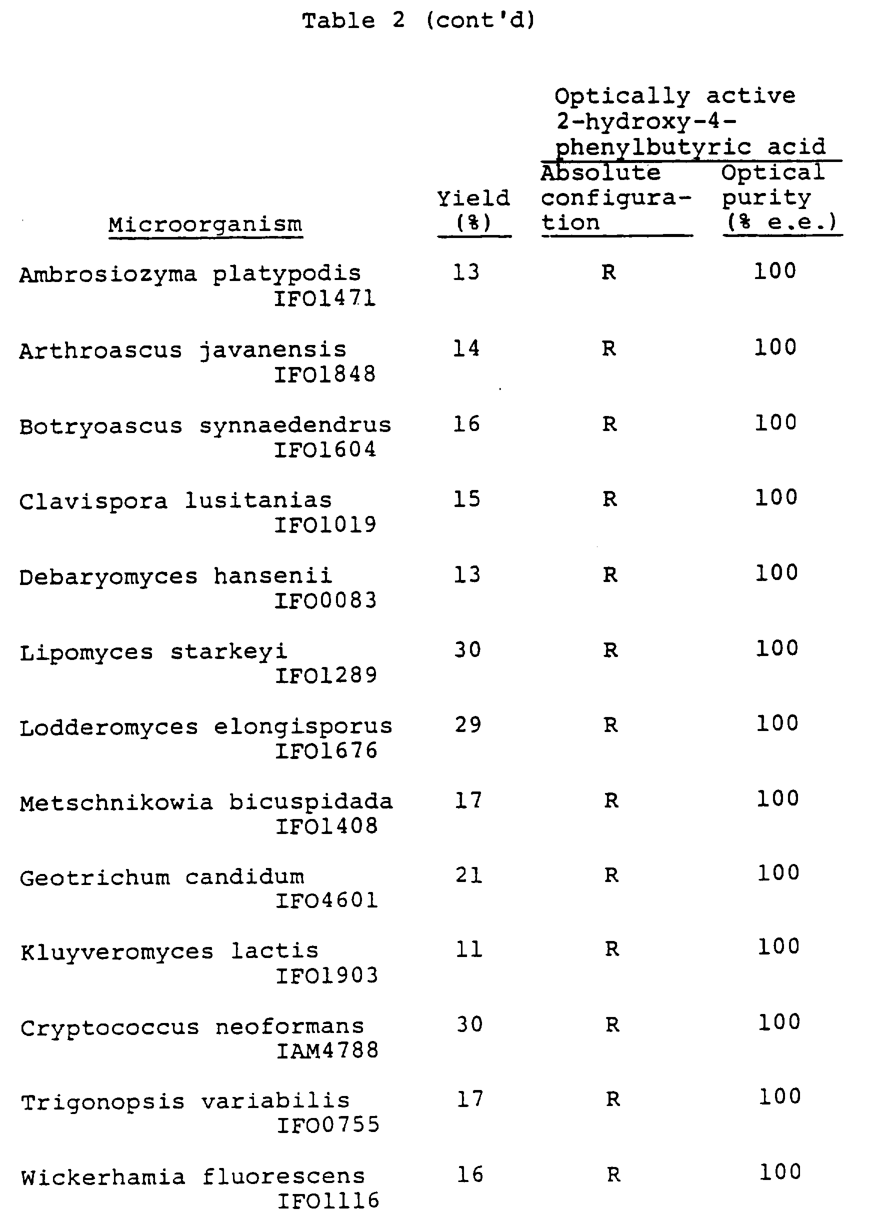

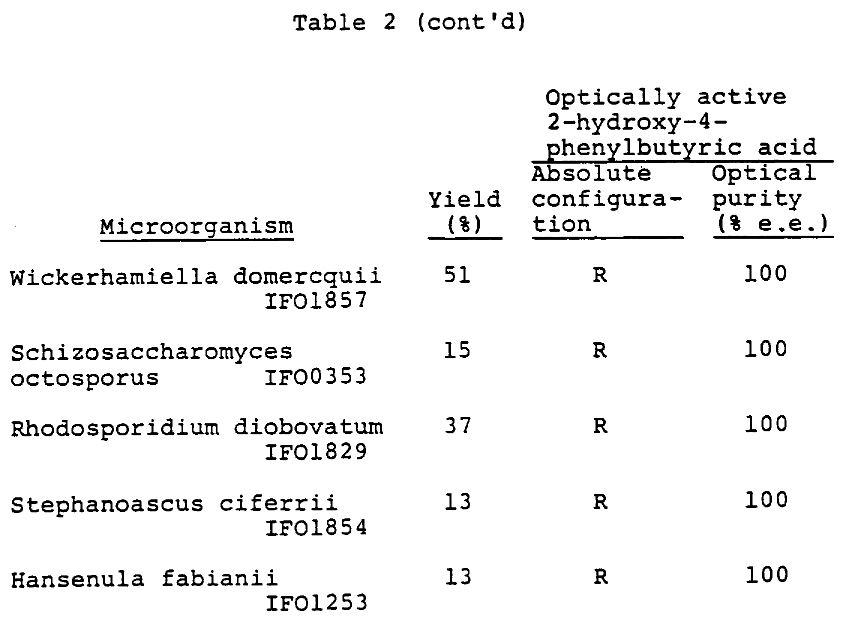

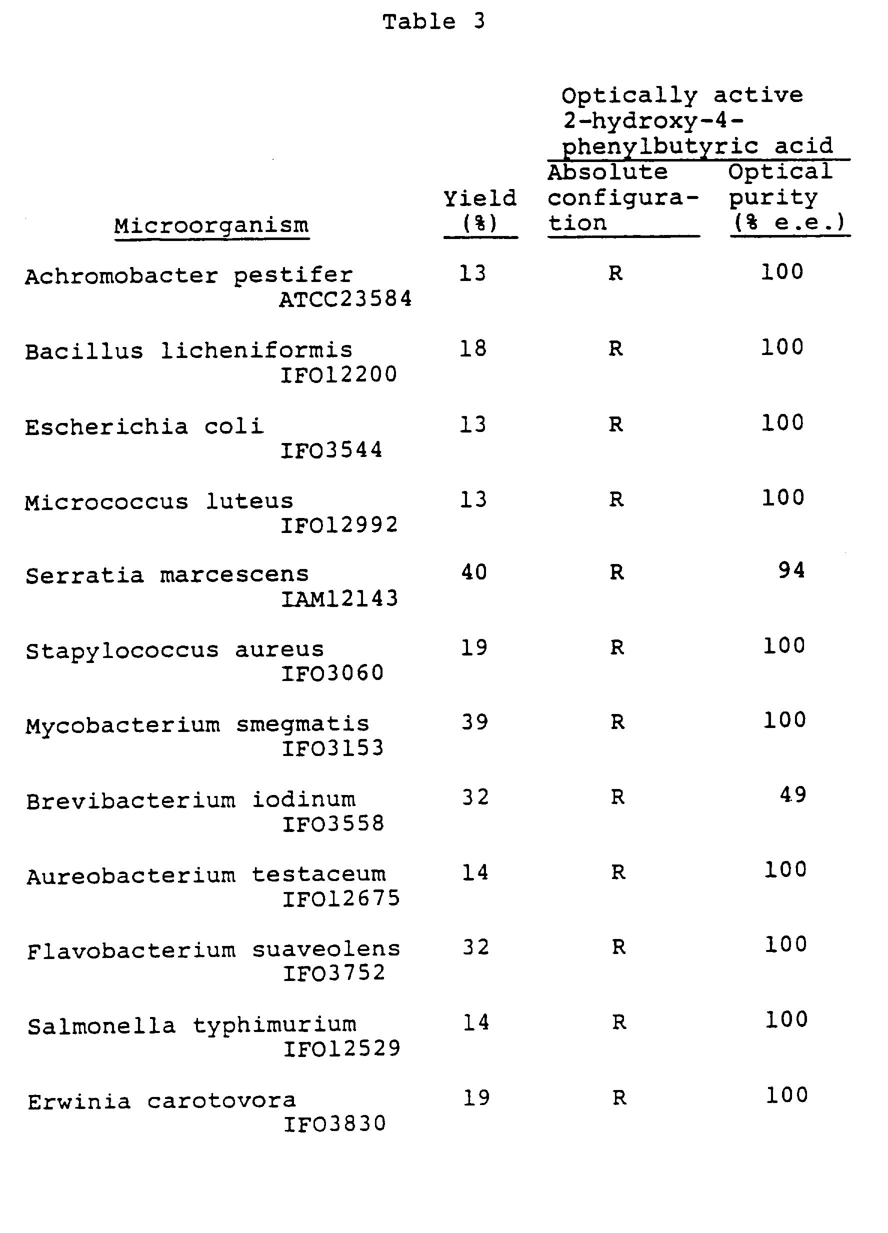

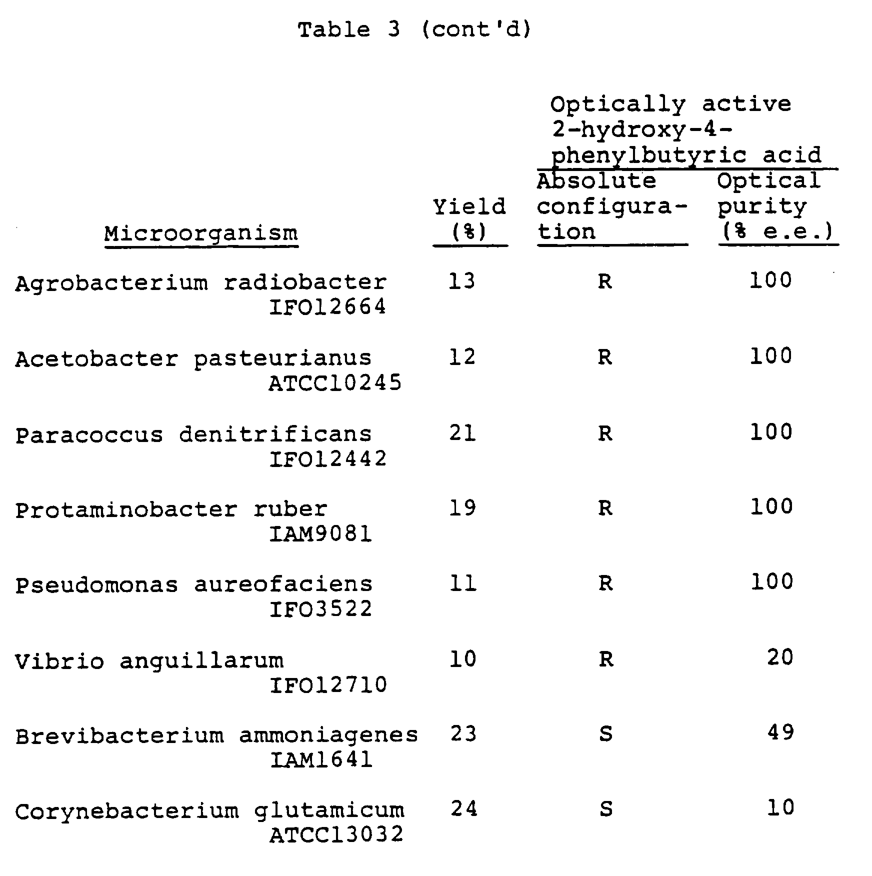

## Claims

1. A process for the production of optically active 2-hydroxy-4-phenylbutyric acid which comprises treating 2-oxo-4-phenylbutyric acid with a microorganism capable of asymmetrically reducing 2-oxo-4-phenylbutyric acid into either (R)-2-hydroxy-4-phenylbutyric acid or (S)-2-hydroxy-4-phenylbutyric acid and collecting the (R)-2-hydroxy-4-phenylbutyric acid or (S)-2-hydroxy-4-phenylbutyric acid thus formed, which microorganism capable of producing (R)-2-hydroxy-4-phenylbutyric acid is selected from among those belonging to the genera Streptococcus, Sporolactobacillus, Pediococcus, Saccharomycopsis, Saccharomyces, Rhodotolura, Candida, Torulaspora, Sporidiobolus, Ambrosiozyma, Arthroascus, Botryoascus, Clavispora, Debaryomyces, Lipomyces, Lodderomyces, Metschnikowia, Geotrichum, Kluyveromyces, Cryptococcus, Trigonopsis, Wickerhamia, Wickerhamiella, Schizosaccharomyces., Rhodosporidium, Stephanoascus, Hansenula, Achromobacter, Bacillus, Escherichia, Micrococcus, Serratia, Staphylococcus, Mycobacterium, Brevibacterium, Chromobacterium, Aureobacterium, Flavobacterium, Salmonella, Erwinia, Agrobacterium, Acetobacter, Paracoccus, Protaminobacter, Pseudomonas, Vibrio, and which microorganism capable of producing (S)-2-hydroxy-4-phenylbutyric acid is selected from among those belonging to the genera Pediococcus, Streptococcus, Brevibacterium and Corynebacterium.

2. The process as claimed in Calim 1 which comprises using a microorganism selected from among those belonging to the genera Streptococcus, Saccharomycopsis, Rhodotolura, Candida, Saccharomyces and Sporidiobolus.

3. The process as claimed in Claim 1 which comprises using a microorganism selected from among those belonging to the genera Sporolactobacillus, Achromobacter, Bacillus, Escherichia, Micrococcus, Serratia, Staphylococcus, Mycobacterium, Chromobacterium, Aureobacterium, Flavobacterium, Salmonella, Erwinia, Agrobacterium, Acetobacter, Paracoccus, Protaminobacter, Pseudomonas, Ambrosiozyma, Arthroascus, Botryoascus, Calvispora, Debaryomyces, Lipomyces, Lodderomyces, Metschnikowia, Geotrichum, Kluyveromyces, Cryptococcus, Trigonopsis, Wickerhamia, Wickerhamiella, Schizosaccharomyces, Rhodosporidium, Stephanoascus and Hansenula.

## Patentansprüche

1. Verfahren für die Herstellung von optisch aktiver 2-Hydroxy-4-phenylbuttersäure, welches das Behandeln von 2-Oxo-4-phenylbuttersäure mit einem Mikroorganismus, welcher fähig ist,2-Oxo-4-phenylbuttersäure zu entweder (R)-2-Hydroxy-4-phenylbuttersäure oder (S)-2-Hydroxy-4-phenylbuttersäure asymmetrisch zu reduzieren, und das Gewinnen der so gebildeten (R)-2-Hydroxy-4-phenylbuttersäure oder (S)-2-Hydroxy-4-phenylbuttersäure umfaßt, wobei der Mikroorganismus, welcher fähig ist, (R)-2-Hydroxy-4-phenylbuttersäure zu produzieren, ausgewählt wird aus solchen, die zu den Gattungen Streptococcus, Sporolactobacillus, Pediococcus, Saccharomycopsis, Saccharomyces, Rhodotolura, Candida, Torulaspora, Sporidiobolus, Ambrosiozyma, Arthroascus, Botryoascus, Clavispora, Debaryomyces, Lipomyces, Lodderomyces, Metschnikowia, Geotrichum, Kluyveromyces, Cryptococcus, Trigonopsis, Wickerhamia, Wickerhamiella, Schizosaccharomyces, Rhodosporidium, Stephanoascus, Hansenula, Achromobacter, Bacillus, Escherichia, Micrococcus, Serratia, Staphylococcus, Mycobacterium, Brevibacterium, Chromobacterium, Aureobacterium, Flavobacterium, Salmonella, Erwinia, Agrobacterium, Acetobacter, Paracoccus, Protaminobacter, Pseudomonas, Vibrio gehören und wobei der Mikroorganismus, welcher fähig ist, (S)-2-Hydroxy-4-phenylbuttersäure zu produzieren, ausgewählt wird aus solchen, die zu den Gattungen Pediococcus, Streptococcus, Brevibacterium und Corynebacterium gehören.

2. Verfahren nach Anspruch 1, welches die Verwendung eines Mikroorganismus, ausgewählt aus solchen, die zu den Gattungen Streptococcus, Saccharomycopsis, Rhodotolura, Candida, Saccharomyces und Sporidiobolus gehören, umfaßt.

3. Verfahren nach Anspruch 1, welches die Verwendung eines Mikroorganismus, ausgewählt aus solchen, die zu den Gattungen Sporolactobacillus, Achromobacter, Bacillus, Escherichia, Micrococcus, Serratia, Staphylococcus, Mycobacterium, Chromobacterium, Aureobacterium, Flavobacterium, Salmonella, Erwinia, Agrobacterium, Acetobacter, Paracoccus, Protaminobacter, Pseudomonas, Ambrosiozyma, Arthroascus, Botryoascus, Clavispora, Debaryomyces, Lipomyces, Lodderomyces, Metschnikowia, Geotrichum, Kluyveromyces, Cryptococcus, Trigonopsis, Wickerhamia, Wickerhamiella, Schizosaccharomyces, Rhodosporidium, Stephanoascus und Hansenula gehören, umfaßt.

## Revendications

1. Procédé pour la production d'acide 2-hydroxy-4-phénylbutyrique optiquement actif, qui comprend le traitement de l'acide 2-oxo-4-phénylbutyrique avec un micro-organisme capable de réduire asymétriquement l'acide 2-oxo-4-phénylbutyrique, soit en acide (R)-2-hydroxy-4-phénylbutyrique, soit en acide (S)-2-hydroxy-4-phénylbutyrique et la récolte de l'acide (R)-2-hydroxy-4-phénylbutyrique ou de l'acide (S)-2-hydroxy-4-phénylbutyrique ainsi formé, lequel micro-organisme capable de produire l'acide (R)-2-hydroxy-4-phénylbutyrique est choisi parmi ceux appartenant aux genres Streptococcus, Sporolactobacillus, Pediococcus, Saccharomycopsis, Saccharomyces, Rhodotolura, Candida, Torulaspora, Sporidiobolus, Ambrosiozyma, Arthroascus, Botryoascus, Clavispora, Debaryomyces, Lipomyces, Lodderomyces, Metschnikowia, Geotrichum, Kluyveromyces, Cryptococcus, Trigonopsis, Wickerhamia, Wickerhamiella, Schizosaccharomyces, Rhodosporidium, Stephanoascus, Hansenula, Achromobacter, Bacillus, Escherichia, Micrococcus, Serratia, Staphylococcus, Mycobacterium, Brevibacterium, Chromobacterium, Aureobacterium, Flavobacterium, Salmonella, Erwinia, Agrobacterium, Acetobacter, Paracoccus, Protaminobacter, Pseudomonas, Vibrio et lequel micro-organisme capable de produire l'acide (S)-2-hydroxy-4-phénylbutyrique est choisi parmi ceux appartenant aux genres Pediococcus, Streptococcus, Brevibacterium et Corynebacterium

2. Procédé selon la revendication 1 qui comprend l'utilisation d'un micro-organisme choisi parmi ceux appartenant aux genres Streptococcus, Saccharomycopsis, Rhodotolura, Candida, Saccharomyces et Sporidiobolus

3. Procédé selon la revendication 1 qui comprend l'utilisation d'un micro-organisme choisi parmi ceux appartenant aux genres Sporolactobacillus, Achromobacter, Bacillus, Escherichia, Micrococcus, Serratia, Staphylococcus, Mycobacterium, Chromobacterium, Aureobacterium, Flavobacterium, Salmonella, Erwinia, Agrobacterium, Acetobacter, Paracoccus, Protaminobacter, Pseudomonas, Ambrosiozyma, Arthroascus, Botryoascus, Clavispora , Debaryomyces, Lipomyces, Lodderomyces, Metschnikowia, Geotrichum, Kluyveromyces, Cryptococcus, Trigonopsis, Wickerhamia, Wickerhamiella, Schizosaccharomyces, Rhodosporidium, Stephanoascus et Hansenula.
